(19) Europäisches Patentamt
European Patent Office
Office européen des brevets

(11) Publication number: **0 255 343 A2**

(12) # EUROPEAN PATENT APPLICATION

(21) Application number: 87306665.8

(22) Date of filing: 28.07.87

(51) Int. Cl.4: C 07 C 103/50
A 61 K 7/24, A 23 L 1/236

(30) Priority: 01.08.86 JP 179886/86

(43) Date of publication of application:
03.02.88 Bulletin 88/05

(84) Designated Contracting States:
CH DE FR GB IT LI NL

(71) Applicant: Takasago Perfumery Co., Ltd.
No. 19-22, Takanawa 3-chome
Minato-ku Tokyo (JP)

(72) Inventor: Yuasa, Yoshifuma c/o Takasago Perfumery Co. Ltd.
Kamata Div. 36-31, Kamata 5-chome
Ohta-ku Tokyo (JP)

Okeda, Yoshiki c/o Takasago Perfumery Co. Ltd.
Kamata Div. 36-31, Kamata 5-chome
Ohta-ku Tokyo (JP)

Tachikawa, Akio c/o Takasago Perfumery Co. Ltd.
Kamata Div. 36-31, Kamata 5-chome
Ohta-ku Tokyo (JP)

Nagakura, Akira c/o Takasago Perfumery Co. Ltd.
Kamata Div. 36-31, Kamata 5-chome
Ohta-ku Tokyo (JP)

Tsuruta, Haruki c/o Takasago Perfumery Co. Ltd.
Kamata Div. 36-31, Kamata 5-chome
Ohta-ku Tokyo (JP)

(74) Representative: Diamond, Bryan Clive et al
Gee & Co. Chancery House Chancery Lane
London WC2A 1QU (GB)

(54) **L-aspartylfenchylamino alcohol derivatives.**

(57) 3-N-(L-Aspartyl)amino-1-fenchyl-2-butanol or 3-N-(L-aspartyl)amino-4-fenchyl-2-butanol are of the formula:

```
      CH2COOH            R1
      |                  |
H2N - CH - C - NH - CH - CH - R2    (I)
           ||            |
           O             OH
```

wherein $R_1$ is a methyl group and $R_2$ is a homofenchyl group, or $R_1$ is homofenchyl and $R_2$ is methyl.

Synthesis is by converting l-fenchone to fenchylaldehyde, which is converted by one of two routes to one of the two isomers of fenchylamino alcohol, and the isomer is reacted with monobenzyl-L-aspartate having a protective group (e.g. carbonbenzoxy), and the protective group is then removed.

The compounds have excellent sweetness characteristics and are useful as a sweetener which has good stability in acidic aqueous solutions.

EP 0 255 343 A2

**Description**

## L-ASPARTYLFENCHYLAMINO ALCOHOL DERIVATIVES

The present invention relates to two novel L-aspartylfenchylamino alcohol derivatives which have excellent sweetness characteristics and which can be widely used, e.g. in foods, or pharmaceuticals as a sweetener of excellent stability in an acidic aqueous solution.

While sucrose is most widely employed as a suitable sweetener, sugars such as fructose, isomerized sugar, glucose, etc., sugar alcohols such as sorbitol, mannitol, etc., natural sweetening substances such as glycyrrhizin, stevioside, thaumatin, etc., and artificial sweetening substances such as saccharin sodium, sodium cyclamate, aspartame, etc. have also been used as sweeteners.

In recent years, there have been an increasing number of patients suffering from caries as well as obesity, diabetes, cardiopathy, hypertension, kidney diseases, etc. mainly resulted from excess calorie intake. Therefore, for such patients or for the maintenance of good health, low calorie sweeteners have been sought and attempted and some have appeared as commercial products on the market. However, the artificial sweeteners are under various restrictions due to their possible toxicity, whereas the natural sweeteners not only have problems with the quality of sweetness, aftertaste, etc. but also are expensive and, therefore, there are not available satisfactory products.

Recently, L-aspartyl-L-phenylalanine methyl ester (hereinafter referred to as APM) has been permitted for use and now is becoming a widely used artificial sweetener.

As regards dipeptides represented by the APM, 500 or more kinds thereof have been reported by Ariyoshi, Kagaku Sosetsu, No. 14, 85-128 (1976); Iwamura, J. Med. Chem., 24, 572-583 (1981); and A. Van der Heijden et al., Chemical Senses and Flavor, 4(2), 141-151 (1979), but those having a degree of sweetness of 500 times or more that of sucrose are very few. Further, since these dipeptides are esters and, hence, undergo hydrolysis in aqueous solutions or a dealcoholation reaction with the amino group of the aspartic acid residue and convert to a diketopiperazine derivative, they often lose their sweetness or are denaturated.

Of dipeptide esters which have been reported so far, that having the greatest degree of sweetness is L-aspartyl-DL-aminomalonic acid methyl fenchyl diester (see Japanese Patent Publication No. 34622/77; Japanese Patent Application (OPI) No. 30566/74; and Chem. Pharm. Bull., 24 (9), 2112 (1976)), but its stability is lower than APM at 80°C and at a pH of 4. (The term "OPI" as used herein refers to a published unexamined Japanese patent applica tion .) On the other hand, in order to solve these problems, there have recently been reported branched-chain amides of L-aspartyl-D-amino acid (see Japanese Patent Application (OPI) No. 127339/81) and (S)-3-amino-4-[(s,s)-1-(1-hydroxyethyl)alkylamino]-4-oxobutyric acid compounds (see U.S. Patent 4,423,029). But they have from 200 to 300 times degree of sweetness and are not completely satisfactory.

It has been reported that L-aspartylaminomalonic acid diesters and L-aspartyl-D-alanine esters, in paticular, esters with fenchyl alcohol, the latter having been proposed by the present inventors in Japanese Patent Applications (OPI) Nos. 200999/86, 291596/86 and 291597/86, generally exhibit a high degree of sweetness.

Further, based on the belief that by converting this ester part to another functional group such as, for example, a hydroxyl group, a compound excellent in both degree of sweetness and stability may be obtained, intensive studies have been made, and as a result, we have discovered that novel L-aspartylfencylamino alcohol derivatives made by condensing an amino alcohol derivative having a fenchyl group and L-aspartic acid not only have a high degree of sweetness and excellent sweetness characteristics, but also are excellent in both solubility in water and stability in acidic aqueous solutions, whereby the present invention has now been accomplished.

An object of the present invention is to provide a sweetening compound which is similar in quality of sweetness to sucrose, is high in degree of sweetness, is low in calories, and which is stable in acidic aqueous solutions.

The present invention provides 3-N-(L-aspartyl)amino-1-fenchyl-2-butanol or 3-N-(L-aspartyl)-amino-4-fenchyl-2-butanol of the following formula (I):

```
        CH2COOH         R1
        |               |
H2N - CH - C - NH - CH - CH - R2    (I)
           ||            |
           O             OH
```

wherein $R_1$ represents a methyl group and then $R_2$ represents a homofenchyl group, or $R_1$ represents a homofenchyl group and then $R_2$ represents a methyl group.

In the accompanying drawing:

Fig. 1 is a graph showing the stability of aqueous solutions of the compounds of the present invention and comparison compounds at pH 4.0 to 80°C.

A process for the production of the compounds of the invention is given below.

1-Fenchone is converted to homofenchylaldehyde which is then converted into one of two fenchylamino

alcohol derivatives, and each of the fenchylamino alcohol derivatives is reacted with monobenzyl Z-L-aspartate and reduced to obtain a compound of the present invention.

These reaction steps are described below in more detail with reference to the formulae.

(i)Synthesis of Homofenchylaldehyde:

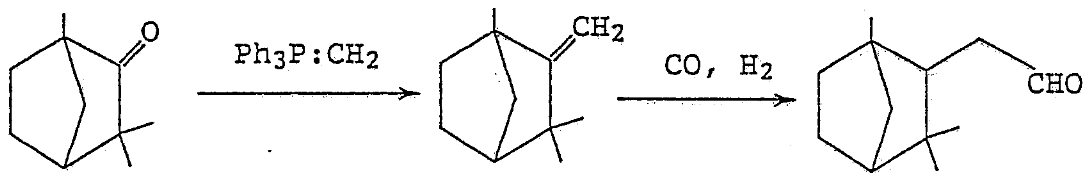


(II) (III)

A Wittig reaction in which ℓ-fenchone is reacted with methylenetriphenylphosphine ($pH_3P:CH_2$) is performed to convert the ℓ-fenchone to an alkene (II). The alkene (II) is reacted with carbon monoxide and hydrogen in the presence of, as a catalyst, cobalt carbonyl by an oxo reaction to prepare homofenchylaldehyde (III).

(ii) Synthesis of Fenchylamino Alcohol:

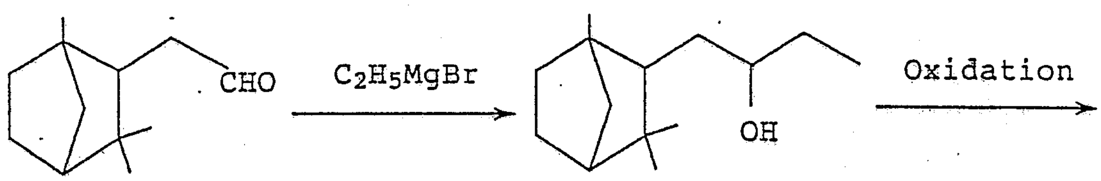


(III) (IV)

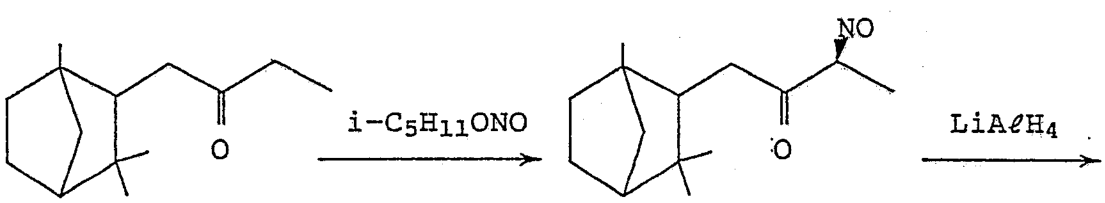


(V) (VI)

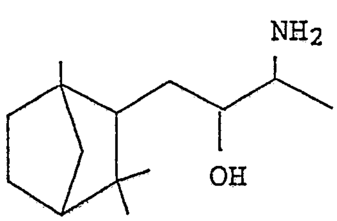

(VII)

The homofenchylaldehyde (III) is subjected to a Grignard reaction using ethylmagnesium bromide to convert it to an alcohol (IV) which is then treated with sodium bichromate in concentrated sulfuric acid to make

into a ketone (V). The ketone (V) is nitrosoficated using isoamyl nitrite, separated by column chromatography, and purified to make into a nitroso ketone (VI). Further, the nitroso ketone (VI) is subjected to reduction with lithium aluminum hydride to produce fenchylamino alcohol (VII).

(iii) Synthesis of Fenchylamino Alcohol Isomer:

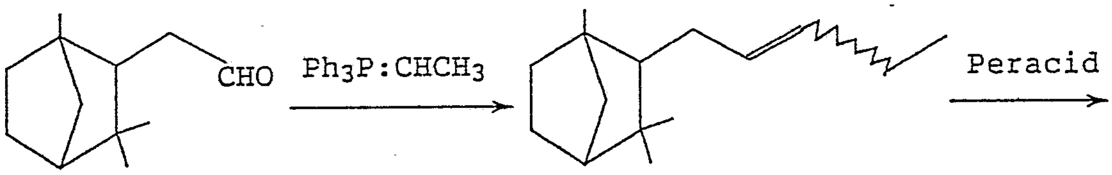


(III) (VIII)

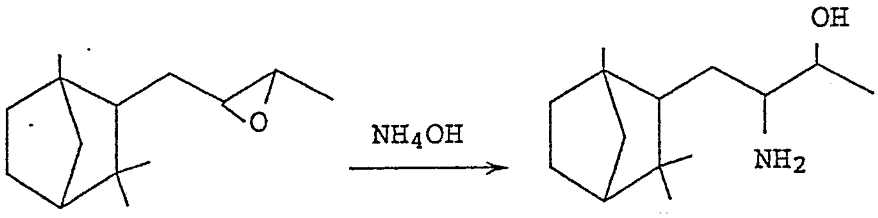


(IX) (X)

The homofenchylaldehyde (III) is reacted with ethylidenetriphenylphosphine ($Ph_3P:CHCH_3$) to convert it to an alkene (VIII) which is reacted with a peracid to make an epoxy compound (IX). Finally, ammonolysis with ammonia water is performed to selectively produce fenchylamino alcohol (X).

(iv) Synthesis of 3-N-(L-Aspartyl)amino-1-(or 4-)fenchyl-2-butanol:

The thus produced fenchylamino alcohol (VII) or its isomer (X) is reacted with monobenzyl Z-L-aspartate (XI) to convert it to a Z-β-benzyl-L-amino alcohol (XII) or (XII′) which is then reduced to remove the protective group (Z) to produce 3-N-(L-aspartyl)amino-1-(or 4-)-fenchyl-2-butanol (Ia) or (Ib).

A preferred example of the protective group Z on the aspartate is a carbobenzoxy group.

These two reaction routes are shown as follows:

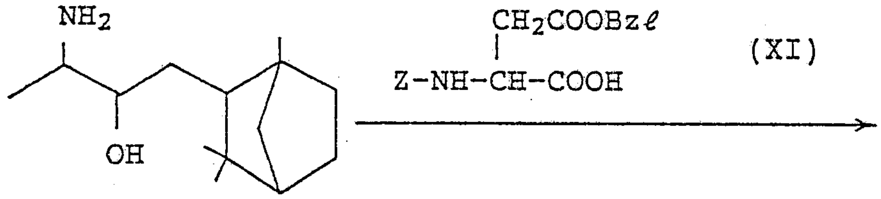

(XI)

(VII)

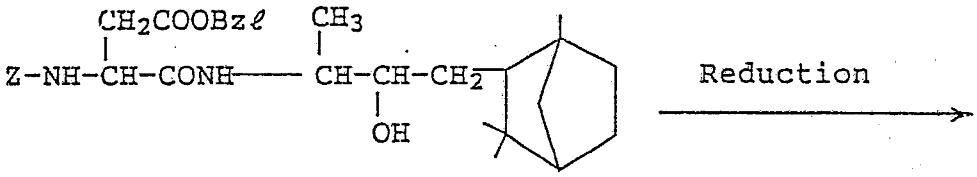


(XII)

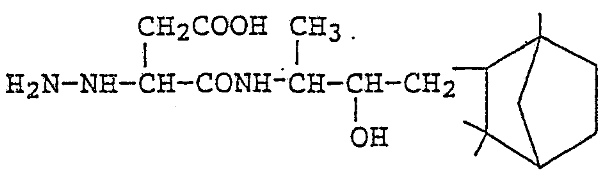

(Ia)

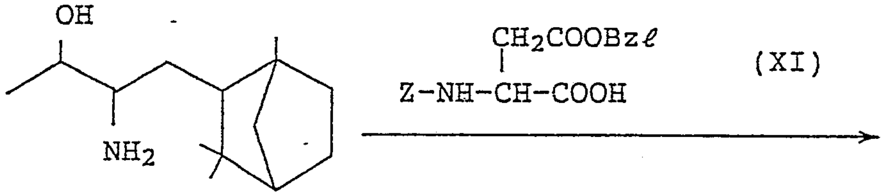

(XI)

(X)

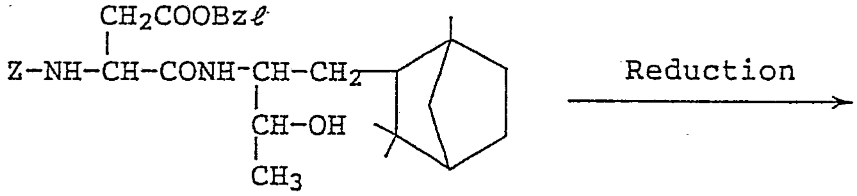


(XII)

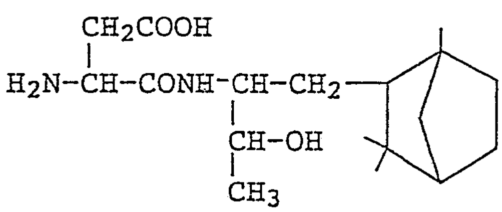

(Ib)

The thus obtained 3-N-(L-aspartyl)amino-1-fenchyl-2-butanol and 3-N-(L-aspartyl)amino-4-fenchyl-2-butanol of the present invention are each a colorless and odorless powder which is readily soluble in water. A diluted aqueous solution thereof exhibits sweetness characteristics extremely similar to those of sucrose and gives almost no disagreeable tastes such as bitter taste, bad taste, and unpleasant aftertaste. In particular, the features of the compounds of the present invention reside in that they are excellent in stability in acidic aqueous solutions and good in heat stability.

The compounds of the present invention may be widely used in various foods, beverages, toothpastes, cigarettes, and cosmetics to which sweeteners are generally added, regardless of shapes. For example, they may be used for soft drinks (such as fruit juice, fruit drinks, and soda pop), lactic acid beverages and carbonated beverages inclusive of their powdered drinks, alcoholic beverages (such as rich wine (sake), synthetic rice wine, fruit liquors, and sweetened rice wine for seasoning (mirin)), cold sweets (such as ice cream and sherbet), fruits in syrup, seasonings (such as miso paste, soy sauce, sauce, vinegar, dressing, mayonnaise, and ketchup), cakes (such as rice cakes, bread, cakes, biscuits, and crackers), chocolates, chewing gum, jelly, sweet jelly of beans, jam marmalade, modified powdered milk, various seaweeds or shellfishes boiled in sweetened soy sauce, canned foods, delicacies of domestic animal meat, edible meats (such as bacon, ham and sausage), fishmeat products (such as boiled fish paste on a board and boiled fish paste on a rod), compound seasonings, lipsticks and oral drugs.

The compounds of the present invention can be used in a powder form as they are or by using appropriate means, for example, by making into a solution by using an appropriate solvent, and the amount to be added can be suitably selected according to the intended use, the subject to be used, the adding means, the kinds and amounts of other sweeteners and seasonings to be used in combination therewith, and the like.

The present invention is hereinafter described in detail with reference to the following Examples and Use Examples.

EXAMPLE 1

Synthesis of 1,3,3-Trimethyl-2-methylenebicyclo[2,2,1]-heptane (II)

50 g (0.14 mole) of methyltriphenylphosphonium bromide was added to a mixed solution of 90 ml of n-butyllithium (15% hexane solution) and 200 ml of diethyl ether over 20 minutes at room temperature under anhydrous conditions. Thereafter, stirring was continued at room temperature for 4 hours and, then, 21.4 g (0.14 mole) of ℓ-fenchone was added dropwise thereto, followed by heating under stirring for a whole day. The reaction mixture was allowed to return to room temperature, and the formed triphenylphosphine oxide was separated off by filtration. 120 ml of water was added to the filtrate, and the organic layer was extracted. The extract was dried over anhydrous Glauber salt, and the solvent was distilled off. The residue was distilled under reduced pressure to obtain 11.6 g (55% in theoretical yield (hereafter referred to simply as "yield") of an alkene as a colorless oil having a boiling point of 82 to 83°C/55 mmHg.

EXAMPLE 2

Synthesis of Homofenchylaldehyde (III)

21 g (0.14 mole) of the alkene obtained in Example 1 was dissolved in 40 ml of benzene in a 500-ml autoclave. After adding thereto a mixture (1:1) of 0.4 ml of pyridine and 0.55 g (1.5 mmoles) of octacarbonyl-2-cobalt, water gas was introduced thereinto under a pressure of 70 atms., and the mixture was allowed to reaot under stirring at 120°C for 5 hours. After allowing the reaction vessel to return to room temperature, the solvent was distilled off under reduced pressure, and distillation was subsequently effected to obtain 12.8 g (yield: 51%) of the desired aldehyde having a boiling point of 70 to 75°C/1 mmHg.

EXAMPLE 3

Synthesis of 1-Fenchyl-2-butanol (IV)

4.9 g (0.2 mole) of magnesium and 21.8 g (0.2 mole) of ethyl bromide were reacted in 100 ml of diethyl ether in a nitrogen gas under anhydrous conditions to produce a Grignard reagent. 12.0 g (67 mmoles) of the homofenchylaldehyde obtained in Example 2 was added dropwise thereto at room temperature, and the mixture was stirred overnight as it stood. The reaction mixture was poured into 100 ml of an ice saturated ammonium chloride aqueous solution, and the ether layer was separated, washed with water, and dried over anhydrous Glauber salt. The ether was distilled off to obtain 11.7 g (yield: 83%) of a colorless oil of 1-fenchyl-2-butanol.

EXAMPLE 4

Synthesis of 1-Fenchyl-2-butanone (V)

11.0 g (52.4 mmoles) of the 1-fenchyl-2-butanol obtained in Example 3 was dissolved in 50 ml of diethyl ether, and 26 ml of an aqueous solution of 5.2 g (17.5 mmoles of sodium bichromate dissolved in 7 ml of concentrated sulfuric acid was added thereto. The mixture was stirred at room temperature for 2 hours, 50 ml of ice water was added, the ether layer was separated, and the aqueous layer was further extracted with 50 ml of diethyl ether. The extracts were combined, washed with water, and dried over anhydrous magnesium sulfate, and the ether was then distilled off. The residue was distilled under reduced pressure to obtain 10.0 g (yield: 92%) of 1-fenchyl-2-butanone as a colorless oil having a boiling point of 88 to 93°C/1 mmHg.

EXAMPLE 5

Synthesis of 1-Fenchyl-3-nitroso-2-butanone (VI)

A solution of 5.5 g (47 mmoles) of isoamyl nitrite in 30 ml of methanol was added dropwise to a mixed solution of 9.5 g (45.7) mmoles of the 1-fenchyl-2-butanone obtained in Example 4, 80 ml of methanol, and 17 ml of concentrated hydrochloric acid over 30 minutes at room temperature. The mixture was stirred overnight, and the methanol was distilled off under reduced pressure. To the residue, 100 ml of diethyl ether and 100 ml of water were added to effect the extraction. The ether layer was washed with water and dried over anhydrous Glauber salt. The ether was distilled off, and the residue was separated and purified by silica gel column chromatography. The obtained crude crystals were recrystallized from diethyl ether/n-hexane to obtain 3.35 g (yield: 31%) of 1-fenchyl-3-nitroso-2-butanone as a colorless crystal having a melting point of 120 to 122°C.

EXAMPLE 6

Synthesis of 1-Fenchyl-3-amino-2-butanol (VII)

A solution of 2.37 g (10 mmoles) of the nitrosobutane obtained in Example 5 dissolved in 20 ml of diethyl ether was added dropwise to a suspension of 1.20 g (30 mmoles) of lithium aluminum hydride in 100 ml of anhydrous diethyl ether over 30 minutes with ice cooling. Thereafter, the mixture was stirred at room temperature overnight. 50 ml of water was carefully added dropwise to the reaction mixture with ice cooling,

and the ether layer was separated. The resulting ether layer was washed twice with 30 ml of water and dried over anhydrous magnesium sulfate. The solvent was distilled off under reduced pressure to obtain 1.75 g (yield: 78%) of 1-fenchyl-3-amino-2-butanol as a yellow oil.

EXAMPLE 7

Synthesis of 1-Fenchyl-2-butene (VIII)

22.4 g (60 mmoles of ethyltriphenylphosphine bromide was added to a mixed solution of 39 ml of n-butyllithium (15% hexane solution) and 100 ml of diethyl ether over 30 minutes while stirring with ice cooling under anhydrous conditions. Thereafter, the stirring was continued at room temperature for 4 hours, and 10.8 g (60 mmoles) of the homofenchylaldehyde obtained in Example 2 was added dropwise thereto and refluxed under heat for 5 hours. The reaction mixture was allowed to return to room temperature, and the purified triphenylphosphine oxide was filtered off. 100 ml of water was added to the filtrate, and the ether layer was separated, washed with water, and then dried over anhydrous Glauber salt. The ether layer was distilled off, and the residue was distilled under reduced pressure to obtain 9.10 g (yield: 79%) of 1-fenchyl-2-butene as a colorless oil having a boiling point of 60 to 65°C/0.5 mmHg.

EXAMPLE 8

Synthesis of 1-Fenchyl-2,3-oxobutane (IX)

A mixed solution of 5.7 g (30 mmoles) of the 1-fenchyl-2-butene obtained in Example 7, 3.6 g (44 mmoles) of sodium acetate, and 80 ml of methylene chloride was maintained at 10°C or below, and 6.3 g of 40% peracetic acid was added dropwise thereto. The mixture was stirred at room temperature for 8 hours. After completion of the reaction, 60 ml of water was poured into the reaction mixture, and the methylene chloride layer was separated and washed with a saturated aqueous solution of Mohr's salt. After repeating the water-washing, the residue was dried over anhydrous magnesium sulfate, and the solvent was distilled off to obtain 5.8 g (yield: 93%) of 1-fenchyl-2,3-oxobutane as an oil.

EXAMPLE 9

Synthesis of 4-Fenchyl-3-amino-2-butanol (X)

In an autoclave, 4.16 g (20 mmoles) of the 1-fenchyl-2,3-oxobutane obtained in Example 8 and 45 ml of concentrated ammonia water were reacted at 140°C for 8 hours. The reaction vessel was allowed to return to room temperature, and the reaction mixture was extracted with 100 ml of diethyl ether, washed with water, and dried over anhydrous potassium carbonate. The ether was distilled off under reduced pressure to obtain 4.10 g (yield: 91%) of 4-fenchyl-3-amino-2-butanol as a red oil.

EXAMPLE 10

Synthesis of 3-N-(Carbobenzoxy-β-benzyl-L-aspartyl)amino-1-fenchyl-2-butanol (XII)

2.5 g (7 mmoles) of β-benzyl carbobenzoxy-L-aspartate was dissolved in 60 ml of dioxane, and 1.26 g (7 mmoles) of N-hydroxy-5-norbornene-2,3-dicarboximide was added thereto. 1.60 g (7.7 mmoles) of dicyclocarbodiimide was added to the mixture while stirring with ice cooling. After allowing the mixture to return to room temperature, the stirring was continued for 4 hours. The produced dicyclohexylurea was filtered off, and a solution of 1.58 g (7 mmoles) of the fenchylamino alcohol obtained in Example 6 dissolved in 10 ml of dioxane was added to the filtrate while stirring with ice cooling. After allowing the mixture to return to room temperature, the stirring was continued overnight to complete the reaction. The solvent was distilled off, and the residue was dissolved in 60 ml of ethyl acetate, washed successively with a 10% citric acid aqueous solution, a 4% sodium bicarbonate aqueous solution and saturated salt water, and dried over anhydrous Glauber salt. The solvent was distilled off, and the residue was purified by silica gel column chromatography to obtain 3.59 g (yield: 91%) of the titled compound as a colorless oil.

EXAMPLE 11

Synthesis of 3-N-(L-Aspartyl)amino-1-fenchyl-2-butanol (Ia)

2.82 g (5 mmoles) of the 3-N-(carbobenzoxy-β-benzyl-L-aspartyl)amino-1-fenchyl-2-butanol obtained in Example 10 was dissolved in 40 ml of methanol, and the solution was catalytically hydrogenated in the presence of palladium black under atmospheric pressure at room temperature for 4 hours. The catalyst was filtered off, and the methanol was distilled off under reduced pressure. To this residue was added an appropriate quantity of n-hexane to obtain 1.53 g (yield: 90%) of crystals of the titled compound.

The characteristics values of this compound are as follows:

m.p.: 128 - 133°C

$[\alpha]_D^{20}$ : +7.9° (c=1, methanol)

NMR ($CD_3OD$, δ) :

0.90, 1.02, 1.05. (each 3H, s, fenchyl $C\underline{H}_3$ groups)

1.20 (3H, d, $C\underline{H}_3$ CH•CH NH OH)
2.63 (2H, m, Asp β-$C\underline{H}_2$)
4.02 (1H, m, Asp α-$C\underline{H}$)
MS:
322 (M+-18), 263, 190, 160, 143, 125, 88 (base)

EXAMPLE 12

Synthesis of 3-N-(carbobenzoxy-β-benzyl-L-aspartyl)amino-4-fenchyl-3-butanol (XII')

2.5 g (7 mmoles) of β-benzyl carbobenzoxy-L-aspartate was dissolved in 60 ml of dioxane, and 1.26 g (7 mmoles) of N-hydroxy-5-norbornene-2,3-dicarboximide was added thereto. 1.60 g (7.7 mmoles) of dicyclocarbodiimide was added to the mixture while stirring with ice cooling. After allowing the mixture to return to room temperature, the stirring was continued for 4 hours. The produced dicyclohexylurea was filtered off, and a solution of 1.58 g (7 mmoles) of the fenchylamino alcohol obtained in Example 9 dissolved in 10 ml of dioxane was added to the filtrate while stirring with ice cooling. After allowing the mixture to return to room temperature, the stirring was continued overnight to complete the reaction. The solvent was distilled off, and the residue was dissolved in 60 ml of ethyl acetate, washed successively with a 10% citric acid aqueous solution, a 4% sodium bicarbonate aqueous solution, and saturated salt water, and dried over anhydrous Glauber salt. The solvent was distilled off, and the residue was purified by silica gel column chromatography to obtain 3.67 g (yield: 93%) of the titled compound as a colorless oil.

EXAMPLE 13

Synthesis of 3-N-(L-aspartyl)amino-4-fenchyl-2-butanol (Ib)

2.82 g (5 mmoles) of the 3-N-(carbobenzoxy-β-benzyl-L-aspartyl)amino-1-fenchyl-2-butanol obtained in Example 12 was dissolved in 40 ml of methanol, and the solution was catalytically hydrogenated in the presence of palladium black under atmospheric pressure at room temperature for 4 hours. The catalyst was filtered off, and the methanol was distilled off under reduced pressure. To this residue was added an appropriate quantity of n-hexane to obtain 1.55 g (yield: 91%) of crystals of the titled compound.

The characteristic values of this compound are as follows:
m.p.: 140 - 144°C
$[\alpha]_D^{20}$ : +0.7° (c=1, methanol)
NMR ($CD_3OD$, δ):
0.19, 0.95, 1.02 (each 3H, s, fenchyl $C\underline{H}_3$ groups)
1.18 (3H, d, $C\underline{H}_3$ CH OH)
2.63 (2H, m, Asp β-$C\underline{H}_2$)
4.08 (1H, m, Asp α-$C\underline{H}$)
MS:
322 (M+-18), 263, 190, 171, 142, 125, 88, 70, 44 (base)

EXAMPLE 14

The 3-N-(L-aspartyl)amino-1-fenchyl-2-butanol obtained in Example 11 (hereinafter referred to as Compound A), the 3-N-(L-aspartyl)amino-4-fenchyl-2-butanol obtained in Example 13 (hereinafter referred to as Compound B), APM, and sucrose were dissolved in water, respectively, and their threshold values were determined by a limit method by a panel consisting of 5 skilled flavorists. The results are set forth in Table 1.

TABLE 1

| Sweetener | Taste intensity Threshold Values (%) | Degree of Sweetness (time) |
|---|---|---|
| Compound A | 0.001 | 600 |
| Compound B | 0.0012 | 500 |
| APM | 0.004 | 150 |
| Sucrose | 0.6 | 1 |

EXAMPLE 15

Compound A, Compound B, APM, and L-aspartyl-DL-aminomalonic acid methylfenchyl ester (AMF) as disclosed in Japanese Patent Application (OPI) No. 30566/74 were each dissolved in a 0.1 M phosphate buffer (pH 4.0) at a concentration of 0.2%. The solution was maintained at 80° C, and the percent remaining was measured periodically by using high performance liquid chromatography, whereby the stability was compared. The results are shown in Fig. 1. The compounds of the present invention were superior to the comparative compounds in stability.

For the sake of convenience of use, 1 g of each of the compounds of Example 11 and Example 13 was mixed thoroughly with 99 g of glucose to make each powder sample of a concentration of 1%. The amounts of Compound A and Compound B used are those of the 1% concentration products.

USE EXAMPLE 1

Fruit Juice-free Carbonated Beverage:

Glucose 200 g
Citric acid 1.5 g
Lemon essence 2 ml
Compound A 25 g

Water was added to the above formulation to make the total volume 400 ml, to which was added 1600 ml of carbonated water to prepare a carbonated beverage. The quality of sweetness was good, similar to that of sucrose.

USE EXAMPLE 2

Cold Sweet:

Powdered starch syrup 180 g
Thickener 3 g
Citric acid 2 g
Table salt 0.1 g
Strawberry essence 1 ml
Compound B 40 g

Water was added to the above formulation to make the total 1000 g.

According to the above formulation, a stock solution was prepared and frozen to obtain a strawberry cold sweet. It gave refreshing sweetness and was tasty.

USE EXAMPLE 3

Sherbet:

Compound A 30 g
Powdered starch syrup 210 g
Stabilizer 3 g
Citric acid 1 g
Yellow color No. 5 q.p.
Orange flavor 1 g

Water was added to the above formulation to make the total 1000 g.

According to the above formulation, the blend was placed in a freezer to prepare a sherbet. It was comparable in taste to the ordinary sherbet using refined sugar.

USE EXAMPLE 4

Toothpaste:

Calcium hydrogenphosphate 500 g
Carboxymethyl cellulose 11 g
Sodim laurylsulfate 15 g
Glycerin 250 g
Compound B 1.7 g
Toothpaste flavor 9.5 g
Sodium benzoate 0.5 g

Water was added to the above formulation to make the total 1000 g.

According to the above formulation, the respective components were kneaded in a blender to prepare a toothpaste. When used, it exhibited refreshing sweetness without bitterness, thus giving good results.

The present invention provides novel 3-N-(L-aspartyl)amino-1-fenchyl-2-butanol or 3-N-(L-aspartyl)-amino-4-fenchyl-2-butanol. Since these compounds have a quality of sweetness similar to that of sucrose and a high degree of sweetness and are stable in acidic aqueous solutions, they can be used as a sweetener for many uses without any restriction from the practical viewpoint.

## Claims

1. 3-N-(L-Aspartyl)amino-1-fenchyl-2-butanol or 3-N-(L-aspartyl)amino-4-fenchyl-2-butanol of the following formula (I):

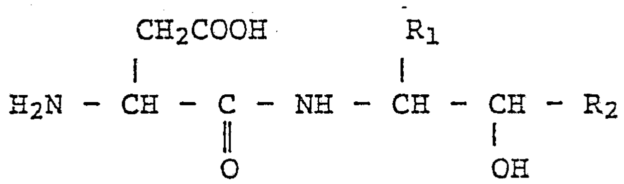

(I)

wherein $R_1$ represents a methyl group and, then, $R_2$ represents a homofenchyl group, or $R_1$ represents a homofenchyl group and, then, $R_2$ represents a methyl group.

2. 3-N-(L-Aspartyl)amino-1-fenchyl-2-butanol.

3. 3-N-(L-Aspartyl)amino-4-fenchyl-2-butanol.

4. A food, beverage, toothpaste, cigarette or cosmetic product containing as sweetener a compound as claimed in Claim 1, 2 or 3.

5. A method of synthesizing a compound as claimed in Claim 1, 2 or 3, wherein homofenchylaldehyde is converted to one of the two isomers of fenchylamino alcohol, which is reacted with a monobenzyl (protective group) -L-aspartate which is then reduced to remove the protective group.

FIG. 1

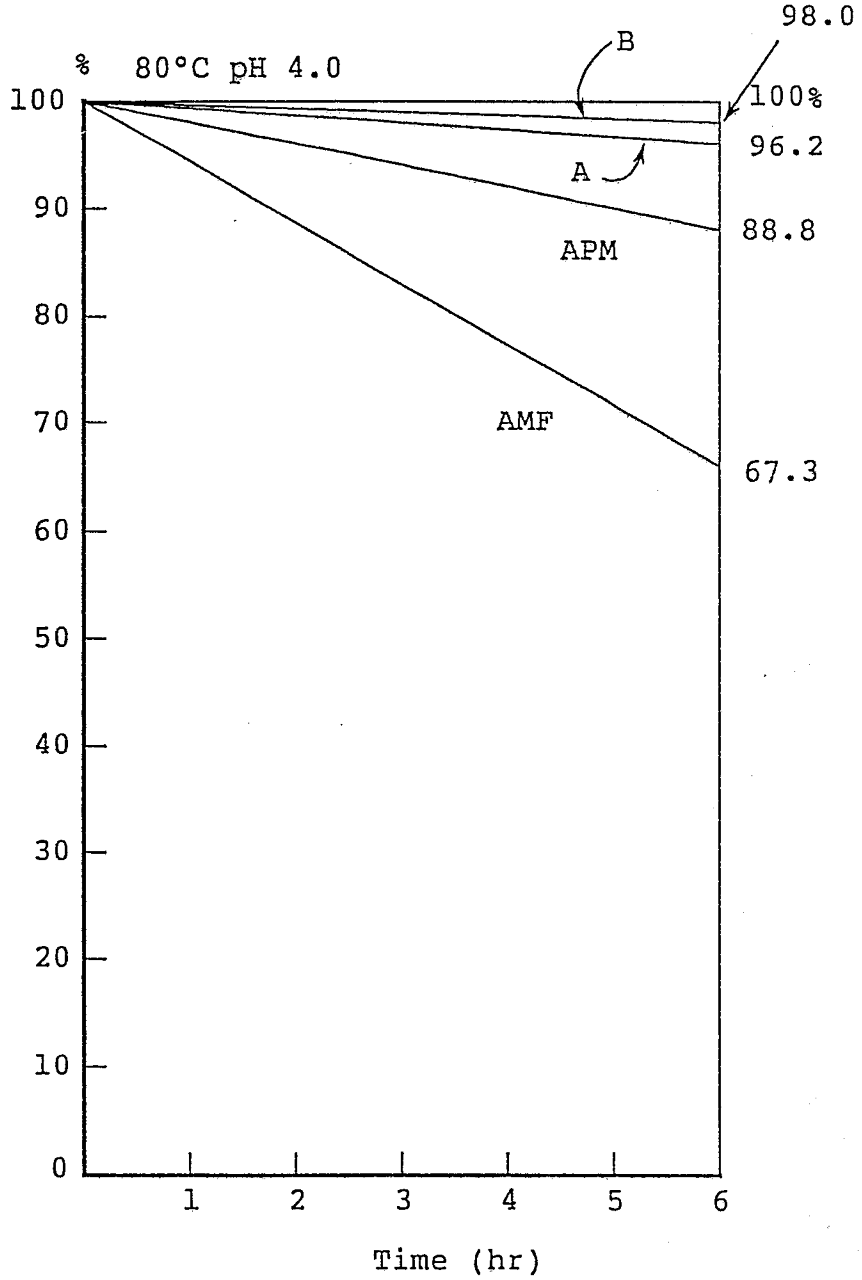

%
80°C pH 4.0
100
90
80
70
60
50
40
30
20
10
0
1
2
3
4
5
6
Time (hr)
B
98.0
100%
96.2
A
88.8
APM
AMF
67.3